(19) Europäisches Patentamt
European Patent Office
Office européen des brevets

(11) EP 4 755 305 A1

(12) EUROPEAN PATENT APPLICATION

(43) Date of publication:
10.06.2026 Bulletin 2026/24

(51) International Patent Classification (IPC):
A61B 6/00 (2024.01)

(21) Application number: 25221003.4

(52) Cooperative Patent Classification (CPC):
A61B 6/544; A61B 6/545

(22) Date of filing: 05.12.2025

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
GE KH LA MA MD TN

(30) Priority: 05.12.2024 KR 20240179446

(71) Applicant: Synicsray
Seoul, Jungnang-gu 02048 (KR)

(72) Inventors:
• AN, Hyo Tai
12059 Namyangju-si (KR)
• CHAE, Won Gi
11902 Guri-si (KR)

(74) Representative: Dragotti & Associati S.P.A.
Via Nino Bixio, 7
20129 Milano (MI) (IT)

(54) **X-RAY DEVICE FOR OPTIMIZING RADIATION EXPOSURE AND X-RAY IMAGING METHOD USING THE SAME**

(57) The present invention relates to an X-ray apparatus. The X-ray apparatus identifies an imaging region based on images generated from an RGB camera and a 3D camera, calculates a thickness of the imaging region using depth map data generated by the 3D camera, and determines values of exposure parameters used during X-ray imaging based on the calculated thickness value, thereby having a technical effect of allowing an appropriate amount of radiation to be irradiated to the imaging region.

**FIG. 1**

EP 4 755 305 A1

**Description**

CROSS-REFERENCE TO RELATED APPLICATION AND CLAIM OF PRIORITY

This application claims the benefit under 35 USC §119 of Korean Patent Application No. 10-2024-00179446, filed on December 5, 2024, in the Korean Intellectual Property Office, the entire disclosure of which is incorporated herein by reference for all purposes.

BACKGROUND

1. FIELD

The present invention relates to an X-ray apparatus and an X-ray imaging method using the same, and more particularly, to an X-ray apparatus capable of obtaining a clear image without excessive radiation exposure by optimizing a radiation dose according to a thickness of a target imaging region, and an X-ray imaging method using the same.

2. BACKGROUND ART

In modern medical diagnostic equipment, X-rays are widely used as non-invasive and rapid diagnostic tools. The radiation dose irradiated during such X-ray imaging must maintain an important balance between patient safety and image quality. Excessive radiation exposure may pose potential risks to patient health, which becomes more serious especially when repeated imaging is required. Conversely, if the radiation dose is insufficient, image quality deteriorates, making accurate diagnosis difficult.

Traditional X-ray apparatuses often irradiate radiation using uniformly set exposure parameters (voltage, current, irradiation time, etc.) without sufficiently considering the thickness or characteristics of the target imaging region. Since this method does not reflect the individual body structure of each patient and the characteristics of the imaging region, it causes problems such as excessive radiation exposure, deterioration of image quality, and increased need for re-imaging.

(Patent Document 1) Korean Patent Publication No. 10-2014-0125502 (October 29, 2014)

SUMMARY

An object of the present invention is to provide an X-ray apparatus capable of capturing an X-ray image by irradiating appropriate radiation to a target imaging region. The present invention is not limited to the technical problems described above, and other technical problems may be derived from the following description.

According to one aspect of the present invention, an X-ray apparatus comprises: an RGB camera configured to generate an RGB image of an imaging region; a 3D camera configured to measure a distance to the imaging region and generate a depth map image; a controller configured to identify the imaging region based on the RGB image and the depth map image, measure a thickness of the identified imaging region, and set an exposure parameter based on the measured thickness of the imaging region; and an X-ray imager configured to generate an X-ray image of the imaging region by irradiating the imaging region using the exposure parameter set by the controller.

In some embodiments, the controller includes a region identifier configured to identify the imaging region and an imaging angle at which the imaging region was imaged from the RGB image and the depth map image, wherein the region identifier determines whether a first imaging region and a first imaging angle identified from the RGB image are identical to a second imaging region and a second imaging angle identified from the depth map image, and when determined to be non-identical, if an identification priority is preset, determines one of the first imaging region and the second imaging region as the imaging region and determines one of the first imaging angle and the second imaging angle as the imaging angle according to the identification priority, and if the identification priority is not preset, generates an alarm and receives input of one of the imaging regions and one of the imaging angles from an operator to determine the imaging region and the imaging angle.

In some embodiments, the X-ray apparatus further comprises a memory storing a region matching table in which examination regions are matched with imaging regions, wherein the region identifier receives information about a target examination region to be examined by the X-ray imaging from an operator, and wherein the region identifier, when the first imaging region and the first imaging angle are determined to be identical to the second imaging region and the second imaging angle, determines whether an imaging region matched to the target examination region is identical to the first imaging region, determines the first imaging region as the imaging region when the imaging region matched to the target examination region is identical to the first imaging region, and generates an alarm and receives input of one of the imaging regions from the operator to determine the imaging region when the imaging region matched to the target examination region is different from the first imaging region.

In some embodiments, the controller includes a thickness calculator configured to measure the thickness of the identified imaging region, wherein the thickness calculator detects a contour of the imaging region from the depth map image and performs thickness measurement on a region inside the detected contour in the depth map image.

In some embodiments, the region matching table further includes thickness measurement region information corresponding to each examination region, and the thickness calculator measures thickness for a partial region among regions inside the contour of the depth map image based on the thickness measurement region information corresponding to the target examination region.

In some embodiments, the memory stores appropriate exposure index (EI) range values matched for each imaging region and patient type, the controller further comprises a re-imaging processor configured to receive the X-ray image of the imaging region generated by the X-ray imager and determine whether re-imaging is necessary, and the re-imaging processor calculates an EI of the generated X-ray image and determines whether re-imaging is necessary by comparing the calculated EI with the appropriate EI range values.

In some embodiments, the memory stores correction coefficients according to EI deviation values for each imaging region in a table format, the controller further comprises a parameter configurator configured to correct the exposure parameter when re-imaging of the X-ray image is determined by the re-imaging processor, and the parameter configurator calculates an EI deviation value between the calculated EI and the appropriate EI range values and corrects the exposure parameter based on the calculated EI deviation value and the determined imaging region.

In some embodiments, the region identifier identifies the imaging region and the imaging angle from the RGB image and the depth map image using an artificial intelligence model trained with a plurality of pre-prepared RGB images and depth map images as input values and labels for imaging regions and imaging angles as output values, and the imaging regions and imaging angles identified from the RGB image and the depth map image by the region identifier are utilized again for training of the artificial intelligence model, thereby continuously improving accuracy of the artificial intelligence model.

According to another aspect of the present invention, an X-ray imaging method performed by a processor comprises: receiving an RGB image and a depth map image of an imaging region; identifying the imaging region based on the RGB image and the depth map image; measuring a thickness of the identified imaging region; setting an exposure parameter based on the measured thickness of the imaging region; and generating an X-ray image of the imaging region by irradiating the imaging region using the set exposure parameter.

**BRIEF DESCRIPTION OF THE DRAWINGS**

FIG. 1 is a configuration diagram of an X-ray apparatus according to an embodiment of the present invention.
FIG. 2 is a schematic flowchart of an X-ray imaging method according to an embodiment of the present invention.

FIG. 3 is a flowchart of some steps of an X-ray imaging method according to an embodiment of the present invention.
FIGS. 4(a) and 4(b) is a diagram illustrating a process for measuring thickness according to an embodiment of the present invention.
FIG. 5 is a table showing EI values for determining whether to re-image according to an embodiment of the present invention.
FIG. 6 is a table showing correction coefficients according to EI deviation values according to an embodiment of the present invention.

**DETAILED DESCRIPTION OF THE EMBODIMENTS**

The following detailed description of the present invention refers to the accompanying drawings, which illustrate, by way of illustration, specific embodiments in which the invention may be practiced. These embodiments are described in sufficient detail to enable those skilled in the art to practice the invention. It should be understood that the various embodiments of the present invention are different but need not be mutually exclusive. For example, specific shapes, structures, and characteristics described herein may be implemented in other embodiments without departing from the spirit and scope of the invention in connection with one embodiment. In addition, it should be understood that the position or arrangement of individual components within each disclosed embodiment may be changed without departing from the spirit and scope of the invention. Therefore, the following detailed description is not to be taken in a limiting sense, and the scope of the invention, if properly described, is limited only by the appended claims, along with the full scope of equivalents to which such claims are entitled. In the drawings, like reference numerals refer to the same or similar functions throughout the several aspects.

Hereinafter, preferred embodiments of the present invention will be described in detail with reference to the accompanying drawings so that those skilled in the art to which the present invention pertains can easily practice the present invention.

Embodiments of the present invention described below relate to an X-ray apparatus capable of optimizing radiation exposure and an X-ray imaging method. Hereinafter, the X-ray apparatus capable of optimizing radiation exposure may be briefly referred to as an "X-ray apparatus," and the X-ray imaging method capable of optimizing radiation exposure may be referred to as an "X-ray imaging method."

In the present embodiment, an imaging region refers to a region that is directly imaged by the X-ray apparatus, that is, an externally visible region of a body. For example, the chest, hand, or knee may be an imaging region. On the other hand, an examination region is a specific structure to be observed or diagnosed using an image captured by X-ray, that is, a part that an examiner wants to see in the captured X-ray image. For example, a doctor may capture an X-ray image of the chest to examine ribs, thoracic vertebrae, or lungs. In this case, the imaging region is the chest, and the examination region may be ribs, thoracic vertebrae, or lungs.

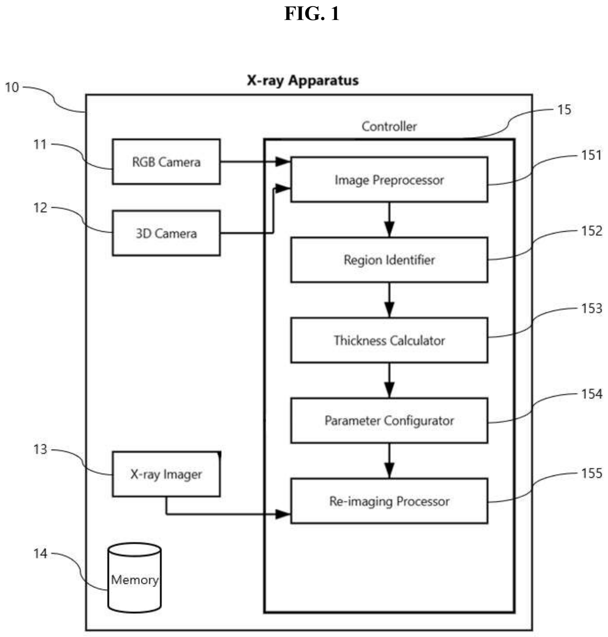

FIG. 1 is a schematic configuration diagram of an X-ray apparatus 10 according to an embodiment of the present invention. Referring to FIG. 1, the X-ray apparatus 10 according to the present embodiment comprises an RGB camera 11, a 3D camera 12, an X-ray imager 13, a memory 14, and a controller 15.

The RGB camera 11 may detect three primary colors of red (R), green (G), and blue (B), process them, and output a digital image expressing a color of the imaging region. That is, the RGB camera 11 may produce an image of the imaging region as viewed by human vision. In the present embodiment, an image of the RGB camera 11 or an RGB image refers to a digital image captured by the RGB camera 11.

The 3D camera 12 is a camera that measures a distance to an imaging target and outputs a result including 3D information. The 3D camera 12 may determine a distance between the camera and the imaging region by emitting light to the imaging region and using a time of flight of the light reflected back from the imaging region, or may determine the distance between the camera and the imaging region from pattern distortion by capturing the imaging region using two lenses.

In the present embodiment, the 3D camera may be any camera capable of measuring a distance to the imaging region, that is, capable of generating a depth map of the imaging region. A depth map generated by the 3D camera 12 is a data representation including information indicating a distance between each pixel of the captured image and the imaging region, and depth map data stores distance information between the imaging region and the camera as a data set. Such depth map data may be expressed as a 2D depth map image by being converted into an RGB image through color mapping. In the present embodiment, an image of the 3D camera 12 refers to a depth map image. In addition, unless otherwise described, a depth map may refer to a depth map image.

Meanwhile, in the present embodiment, the RGB camera 11 and the 3D camera 12 may capture the same region. In addition, although the RGB camera 11 and the 3D camera 12 are illustrated as separate components in FIG. 1, in embodiments of the present invention, the RGB camera 11 and the 3D camera 12 may be implemented as a single camera. That is, both an RGB image and a depth map of the imaging region may be acquired with a single camera.

The X-ray imager 13 may irradiate radiation to the imaging region using an X-ray tube, and a detector may receive radiation transmitted through the imaging region and convert it into a digital signal according to radiation intensity to output an X-ray image. The configuration and operation of the X-ray imager 13 belong to techniques well known to those of ordinary skill in the art to which the present embodiment pertains, and thus detailed description thereof will be omitted to prevent the features of the present embodiment from being obscured.

The memory 14 may store various data necessary for the controller 15 according to the present embodiment to control radiation exposure of the X-ray apparatus 10, such as images captured by the RGB camera 11 and the 3D camera 12, information for determining an exposure parameter from a measured thickness of the imaging region, information for correcting an exposure parameter during re-imaging, and information on imaging regions and examination regions. At this time, information on imaging regions and examination regions may be matched with each other and stored as a region matching table. More specifically, each examination region may be matched and stored with imaging region information that should be captured to confirm each examination region in an X-ray image. For example, the examination region "thoracic vertebrae" may be matched and stored with the imaging region "chest." In addition, each examination region may be matched and stored together with thickness measurement region information. For example, the examination region "thoracic vertebrae" may be matched with a vertical center region, and the examination region "ribs" may be matched with an upper region, so that even for the same chest imaging image, the thickness of the vertical center region of the chest image may be measured during "thoracic vertebrae" examination, and the thickness of the upper region of the chest may be measured during "ribs" examination.

The controller 15 may control radiation irradiation of the X-ray imager 13 based on images captured by the RGB camera 11 and the 3D camera 12. The controller 15 according to the present embodiment comprises an image preprocessor 151, a region identifier 152, a thickness calculator 153, a parameter configurator 154, and a re-imaging processor 155.

The image preprocessor 151 may preprocess the depth map image generated by the 3D camera 12, thereby improving accuracy of operations subsequently performed using the depth map. In embodiments of the present invention, the image preprocessor 151 may preprocess the depth map image through a machine learning-based preprocessing method. More specifically, the image preprocessor 151 may use CNN layers to extract respective features from the image captured by the RGB camera 11 and the depth map image captured by the 3D camera 12, combine the two extracted features, and output a corrected depth map by correcting depth map data based on the combined features. At this time, a loss function $f\_CNN$ may be used to minimize a difference between the corrected depth map and an actual depth map. The corrected depth map may be expressed by the following function: $D\_refined = f\_CNN(I\_RGB, D\_noisy)$ (where $D\_refined$: corrected depth map, $I\_RGB$: input RGB image, D_noisy: actual depth map with noise, f_CNN: correction function learned by the CNN model)

The region identifier 152 may identify the imaging region captured by each camera 11, 12 and an imaging angle at which the imaging region was captured from images generated by the RGB camera 11 and the 3D camera 12. In the present embodiment, the region identifier 152 may identify the imaging region from the RGB image and the depth map image preprocessed by the image preprocessor 151 using machine learning. Such machine learning may use a model trained using a deep learning framework such as PyTorch or Tensor-Flow with pre-prepared RGB camera 11 images and 3D camera 12 images of imaging regions captured under various imaging conditions and imaging angles as input values and labels for imaging regions and imaging angles as output values. In embodiments of the present invention, each image acquired from the RGB camera 11 and the 3D camera 12 and imaging region information identified therefrom may be utilized again as training data, thereby continuously improving accuracy of the trained model.

Meanwhile, in the present embodiment, the imaging region and imaging angle identified from the image of the RGB camera 11 may be different from the imaging region and imaging angle identified from the image of the 3D camera 12. In this case, the region identifier 152 may determine one imaging region and imaging angle according to a predetermined identification priority. For example, if the identification priority is predetermined such that the RGB camera 11 has priority for the imaging region and the 3D camera 12 has priority for the imaging angle, and the imaging region is determined as the chest and the imaging angle is determined as lateral from the RGB image, and the imaging region is determined as the leg and the angle is determined as frontal from the depth map image, the region identifier 152 may determine the imaging region as the chest and the imaging angle as frontal according to the identification priority. Such identification priority may be set differently depending on imaging environments.

In the present embodiment, if the identification priority is not predetermined in a situation where the imaging region and imaging angle identified from the image of the RGB camera 11 are different from the imaging region and imaging angle identified from the image of the 3D camera 12, the region identifier 152 may generate an alarm so that an operator may select the correct imaging region and imaging angle.

Meanwhile, in an embodiment of the present invention, the region identifier 152 may receive target examination region information, which is a target of examination, from an operator in advance. In this case, the region identifier 152 may first identify whether the imaging region and imaging angle identified from the image of the RGB camera 11 match the imaging region and imaging angle identified from the image of the 3D camera 12, and when the identified imaging region and imaging angle match each other, may verify reliability of the identified imaging region based on the target examination region information received in advance. More specifically, the region identifier 152 may acquire an imaging region matched to the target examination region received in advance from the region matching table stored in the memory 14, and may verify reliability of the imaging region identified from the image of the RGB camera 11 and the image of the 3D camera 12 by comparing the acquired imaging region with the imaging region identified from the image of the RGB camera 11 and the image of the 3D camera 12 to determine whether they match. If the two imaging regions are different, the region identifier 152 may generate an alarm so that an operator may select the correct imaging region and imaging angle.

The thickness calculator 153 may measure a thickness of the imaging region based on the imaging region and imaging angle identified by the region identifier 152. In the present embodiment, the thickness calculator 153 may measure the thickness of the imaging region using the depth map image and depth map data generated by the 3D camera 12. The thickness of the measured region measured by the thickness calculator 153 may be expressed as follows: t = SID - SSD (where t: thickness of the imaging region, SID: distance from the X-ray tube to the detector, SSD: distance from the X-ray tube to the surface of the imaging region)

At this time, the distance SSD from the X-ray tube to the surface of the imaging region may be expressed as an average value ($\overline{d}$) of the distance from the X-ray tube to the surface of the imaging region, and the average value ($\overline{d}$) may be calculated by the following Equation 1.

[Equation 1]

$$\overline{d} = \frac{1}{A} \sum_{i=\frac{h}{2}-\frac{b}{2}}^{\frac{h}{2}+\frac{b}{2}-1} \sum_{j=\frac{w}{2}-\frac{a}{2}}^{\frac{w}{2}+\frac{a}{2}-1} D(i,j)$$

where A is an area of a measurement region, a is a horizontal length of the measurement region, b is a vertical length of the measurement region, w is a width of an image, h is a height of the image, and D(i, j) is a matrix expression indicating a distance from a specific pixel in the region to an object, which may be obtained using a ToF camera manufacturer SDK or the like.

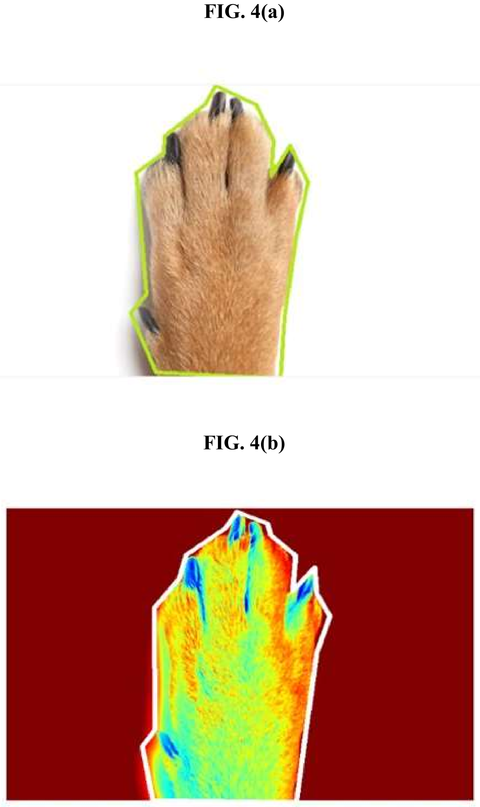

In the present embodiment, the thickness calculator 153 may detect a contour of the imaging region using the depth map image and measure thickness only for a depth map image region inside the detected contour to more accurately perform thickness measurement of the imaging region. That is, the distance from the X-ray tube to the surface of the imaging region may be measured only for pixels inside the contour and used to measure the thickness of the imaging region. This is to prevent the distance between the detector outside the imaging region and the X-ray tube from being reflected in the thickness of the imaging region. If it is difficult to identify the contour of the imaging region from the depth map image, the contour of the imaging region may be detected from the RGB image and then applied to the depth map image. FIGS. 4(a) and 4(b) illustrates detecting a contour of an imaging target from such an RGB image and a depth map image.

Meanwhile, in an embodiment of the present invention, the thickness calculator 153 may determine a thickness measurement region based on target examination region information received in advance. For example, the thickness calculator 153 may acquire thickness measurement region information (i.e., a vertical center region) matched and stored with "thoracic vertebrae" from the memory when the target examination region is "thoracic vertebrae." Thereafter, the thickness calculator 153 may determine a region near a vertical center line that cuts the region left and right among the depth map image region inside the identified contour as a thickness measurement region, and may calculate an average thickness of the vertical center region of the chest as an average thickness of the imaging region. As such, by setting the thickness measurement region more precisely based on the target examination region, the radiation amount may be adjusted more precisely.

The parameter configurator 154 may set values of various exposure parameters based on the thickness of the imaging region measured by the thickness calculator 153. The exposure parameters are various parameters to be used for the X-ray imager 13 to irradiate radiation and capture an X-ray image, and may include a voltage value (kV), a current value (mA), and an irradiation time (ms). In the present embodiment, each exposure parameter may be matched and stored for each thickness of the imaging region in the memory 14, and the parameter configurator 154 may acquire an exposure parameter matched and stored with the imaging region identified by the region identifier 152 and the thickness measured by the thickness calculator 153 from the memory 14 and set it as an exposure value of the X-ray imager.

When the X-ray imager 13 performs X-ray imaging of the imaging region based on the exposure parameter by the parameter configurator 154, the re-imaging processor 155 may receive the captured X-ray image and determine whether re-imaging is necessary based on the received X-ray image.

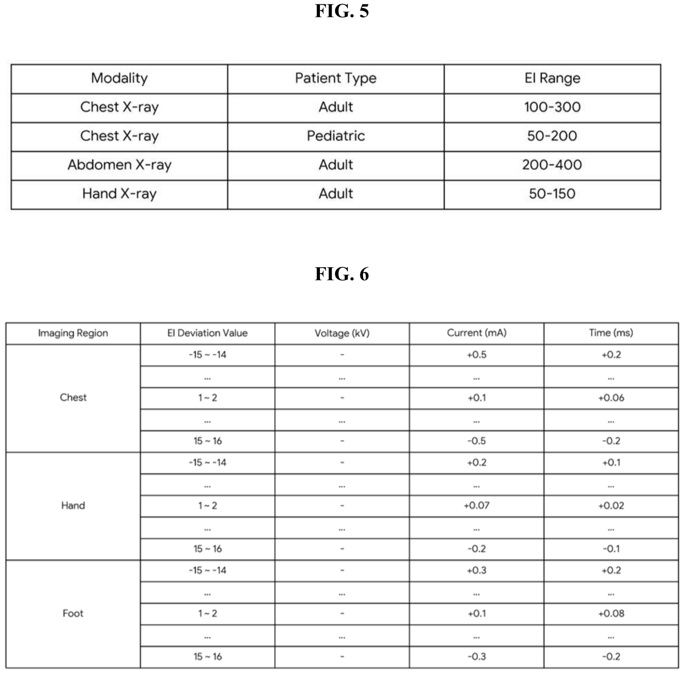

In the present embodiment, the re-imaging processor 155 may calculate an exposure index (EI) from the received X-ray image and determine whether re-imaging is necessary based on the calculated EI value. The re-imaging processor 155 may determine whether re-imaging is necessary by comparing the calculated EI value with an appropriate EI range predetermined and stored in the memory 14. In the present embodiment, as illustrated in FIG. 5, appropriate EI range values may be matched and stored in the memory 14 for each imaging region and patient type (adult or pediatric). The re-imaging processor 155 may determine that re-imaging is not necessary when the calculated EI value is within the stored appropriate EI range value. If the calculated EI value has a value outside the appropriate EI range, the re-imaging processor 155 may determine that re-imaging is necessary and request the parameter configurator 154 to reset the exposure parameter.

In an embodiment of the present invention, the re-imaging processor 155 may determine that re-imaging is necessary when the EI calculated from the received X-ray image deviates from the appropriate EI range by a certain amount or more. That is, the re-imaging processor 155 may calculate a deviation value of the calculated EI and determine whether re-imaging is necessary based on the calculated deviation value. At this time, if the calculated EI has a value smaller than the appropriate EI value range, an EI deviation value is obtained by subtracting a minimum appropriate EI value from the calculated EI value. If the calculated EI has a value larger than the appropriate EI value range, an EI deviation value is obtained by subtracting a maximum appropriate EI value from the calculated EI value. The re-imaging processor 155 may determine not to re-image if an absolute value of the calculated EI deviation value is less than 0.5, may generate an alarm to allow an operator to check the captured X-ray if the absolute value is 0.5 or more and less than 1, and may determine that re-imaging of the X-ray is necessary if the absolute value is 1 or more.

In another embodiment of the present invention, the re-imaging processor 155 may measure quality of the captured X-ray image by analyzing the captured X-ray image using at least one of frequency domain analysis and histogram analysis, and may determine to re-image the X-ray if the measured X-ray image quality is below a predetermined reference value.

The parameter configurator 154 that receives an exposure parameter reset request from the re-imaging processor 155 may correct the exposure parameter based on the calculated EI deviation value. In the present embodiment, as illustrated in FIG. 6, correction coefficients based on EI deviation values for each imaging region may be stored in a table format in the memory 14. For example, assume that the parameter configurator 154 receives an exposure parameter reset request from the re-imaging processor 155, and at this time, an EI deviation value calculated by the re-imaging processor 155 is 1.5, the imaging region is the chest, and the exposure parameters during imaging were voltage 120 kV, current 3.0 mA, and time 10 ms. Referring to FIG. 6, when the imaging region is the chest and the EI deviation value is 1.5, the correction coefficients are that voltage is unchanged (i.e., 0), current is +0.1, and time is +0.06. Accordingly, the parameter configurator 154 may correct the exposure parameters to voltage 120 kV, current 3.1 mA, and time 10.06 ms, respectively, for re-imaging, and may cause the X-ray imager 13 to re-image the X-ray using the corrected exposure parameters. As such correction coefficients are set and stored for each imaging region, the exposure parameters may be corrected to optimal values tailored to characteristics of each imaging region.

Those of ordinary skill in the art to which the present embodiment pertains may understand that these components of the controller may be implemented as hardware that provides specific functions, or may be implemented as a combination of memory, processor, bus, and the like on which software that provides specific functions is recorded. Each of the above-described components is not necessarily implemented as separate hardware, and a plurality of components may be implemented by common hardware, for example, a combination of a processor, memory, bus, and the like.

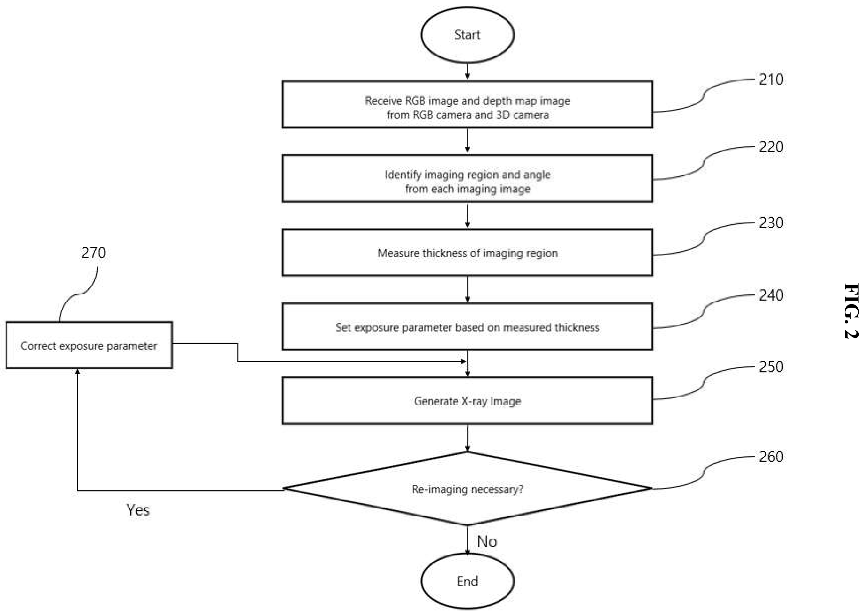

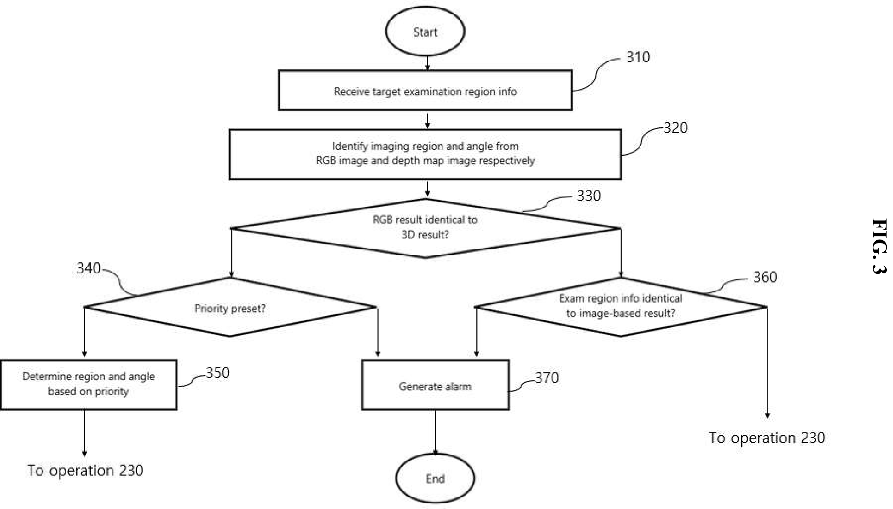

Hereinafter, an X-ray imaging method according to an embodiment of the present invention will be described in detail with reference to FIGS. 2 and 3, which illustrate flowcharts of the X-ray imaging method according to the present embodiment. In operation 210, the region identifier 152 may receive the RGB image generated by the RGB camera 11 and the depth map image generated by the 3D camera 12. At this time, the region identifier 152 may receive the depth map image preprocessed through a machine learning-based preprocessing method as an image generated by the 3D camera 12.

In operation 220, the region identifier 152 may identify the imaging region captured by each camera 11, 12 and the imaging angle at which the imaging region was captured from the RGB image and the depth map image received in operation 210. In the present embodiment, the region identifier 152 may identify the imaging region from the RGB image and the depth map image preprocessed by the image preprocessor 151 using machine learning. FIG. 3 is a diagram illustrating a flowchart of a process of identifying an imaging region and an imaging angle according to an embodiment of the present invention in this operation. Referring to FIG. 3, in operation 310, the region identifier 152 may receive information about a target examination region. Such information about the target examination region may be received from an operator in advance.

In operation 320, the region identifier 152 may identify the imaging region and imaging angle captured by the RGB camera 11 and the 3D camera 12 from the RGB image and the depth map image received in operation 210 using machine learning.

In operation 330, the region identifier 152 may determine whether the imaging region and imaging angle identified from the image of the RGB camera 11 and the imaging region and imaging angle identified from the image of the 3D camera 12 are identical to each other. As a result of the determination, if the imaging region and imaging angle identified from the two images are different from each other, the process proceeds to operation 340, and if they are identical, the process proceeds to operation 360.

In operation 340, the region identifier 152 may determine whether an identification priority is preset and stored. If the identification priority is stored, the imaging region and imaging angle may be determined according to the identification priority (operation 350), and the process proceeds to operation 230 of FIG. 2. If the identification priority is not stored, an alarm may be generated (operation 370) to allow an operator to check the result.

In operation 360, the region identifier 152 may verify reliability of the imaging region identified in operation 320 based on the target examination region information received in operation 310. The region identifier 152 may acquire an imaging region matched to the target examination region received in advance from the memory 14, and may verify reliability of the imaging region identified from the image of the RGB camera 11 and the image of the 3D camera 12 by comparing the acquired imaging region with the imaging region identified from the image of the RGB camera 11 and the image of the 3D camera 12 to determine whether they match. If the imaging region matched to the target examination region is different from the imaging region identified in operation 320, the region identifier 152 may generate an alarm (operation 370) so that an operator may select the correct imaging region and imaging angle, and if the imaging region matched to the target examination region is identical to the imaging region identified in operation 320, the region identifier 152 may determine that the imaging region identified in operation 320 is reliable and proceed to operation 230.

In operation 230, the thickness calculator 153 may measure the thickness of the imaging region based on the imaging region and imaging angle determined in operation 220. In the present embodiment, the thickness calculator 153 may measure the thickness of the imaging region using the depth map image and depth map data generated by the 3D camera 12. At this time, the thickness calculator 153 may detect a contour of the imaging region using the depth map image and measure thickness only for a depth map image region inside the detected contour to more accurately perform thickness measurement of the imaging region.

That is, the distance from the X-ray tube to the surface of the imaging region may be measured only for pixels inside the contour and used to measure the thickness of the imaging region. This is to prevent the distance between the detector outside the imaging region and the X-ray tube from being reflected in the thickness of the imaging region. If it is difficult to identify the contour of the imaging region from the depth map image, the contour of the imaging region may be detected from the RGB image and then applied to the depth map image.

Meanwhile, in an embodiment of the present invention, the thickness calculator 153 may determine a thickness measurement region based on the target examination region information received in operation 310 of FIG. 3. For example, the thickness calculator 153 may acquire thickness measurement region information (i.e., a vertical center region) matched and stored with "thoracic vertebrae" from the memory when the examination region is "thoracic vertebrae." Thereafter, the thickness calculator 153 may determine a region near a vertical center line that cuts the region left and right among the depth map image region inside the identified contour as a thickness measurement region, and may calculate an average thickness of the vertical center region of the chest as an average thickness of the imaging region. As such, by setting the thickness measurement region more precisely based on the target examination region, the radiation amount may be adjusted more precisely.

In operation 240, the parameter configurator 154 may determine an exposure parameter based on the imaging region identified in operation 220 and the thickness of the imaging region calculated in operation 230. More specifically, the parameter configurator 154 may determine an exposure parameter by acquiring exposure parameter values matched and stored for each thickness of each imaging region from the memory 14.

In operation 250, the X-ray imager 13 may perform X-ray imaging using the exposure parameter determined in operation 240.

In operation 260, the re-imaging processor 155 may receive the X-ray image captured in operation 250 and determine whether re-imaging is necessary based on the received X-ray image. In the present embodiment, the re-imaging processor 155 may calculate an EI (Exposure Index) from the received X-ray image and determine whether re-imaging is necessary based on the calculated EI value. The re-imaging processor 155 may determine whether re-imaging is necessary by comparing the calculated EI value with an appropriate EI range predetermined and stored in the memory 14.

The re-imaging processor 155 may determine that re-imaging is not necessary when the calculated EI value is within the stored appropriate EI range value. If the calculated EI value has a value outside the appropriate EI range, the re-imaging processor 155 may determine that re-imaging is necessary and request the parameter configurator 154 to reset the exposure parameter.

Meanwhile, in an embodiment of the present invention, the re-imaging processor 155 may determine that re-imaging is necessary when the EI calculated from the received X-ray image deviates from the appropriate EI range by a certain amount or more. That is, the re-imaging processor 155 may calculate a deviation value of the calculated EI and determine whether re-imaging is necessary based on the calculated deviation value. For example, the re-imaging processor 155 may determine not to re-image if an absolute value of the calculated EI deviation value is less than 0.5, may generate an alarm to allow an operator to check the captured X-ray if the absolute value is 0.5 or more and less than 1, and may determine that re-imaging of the X-ray is necessary if the absolute value is 1 or more. If it is determined by the re-imaging processor 155 that re-imaging is not necessary, the process ends. However, if it is determined by the re-imaging processor 155 that re-imaging is necessary, the process proceeds to operation 270.

In operation 270, the parameter configurator 154 may correct the exposure parameter to re-image the X-ray image. In the present embodiment, the parameter configurator 154 may correct the exposure parameter based on the EI deviation value calculated in operation 260. In the memory 14 according to the present embodiment, correction coefficients based on EI deviation values for each imaging region are stored. Accordingly, when the parameter configurator 154 receives an exposure parameter correction request for re-imaging, the parameter configurator 154 may correct the exposure parameter by acquiring a correction coefficient value matched to the imaging region identified in operation 220 and the EI deviation value calculated in operation 260 from the memory 14. Thereafter, the process returns to operation 250, where the X-ray imager may perform X-ray imaging, and in operation 260, the re-imaging processor 155 may determine whether the re-imaged X-ray image needs to be captured again.

According to the X-ray apparatus 10 according to the present embodiment, by identifying the imaging region based on images generated from the RGB camera 11 and the 3D camera 12, calculating the thickness of the imaging region using depth map data generated by the 3D camera 12, and determining values of exposure parameters used for X-ray imaging based on the calculated thickness value, it is possible to irradiate an appropriate amount of radiation to the imaging region.

The embodiments according to the present invention described above may be implemented in the form of program instructions that may be executed through various computer components and recorded on a computer-readable recording medium. The computer-readable recording medium may include program instructions, data files, data structures, and the like alone or in combination. The program instructions recorded on the computer-readable recording medium may be those specially designed and configured for the present invention or may be known and available to those skilled in the computer software field. Examples of computer-readable recording media include hardware devices specially configured to store and execute program instructions, such as hard disks, ROM, RAM, flash memory, and the like. Examples of program instructions include not only machine language code such as that produced by a compiler but also high-level language code that may be executed by a computer using an interpreter or the like. The hardware device may be configured to operate as one or more software modules to perform processing according to the present invention, and vice versa.

Although the present invention has been described above by specific matters such as specific components and limited embodiments and drawings, this is provided only to help a more general understanding of the present invention, the present invention is not limited to the above embodiments, and those of ordinary skill in the art to which the present invention pertains may make various modifications and variations from such description.

Therefore, the spirit of the present invention should not be limited to the above-described embodiments, and all things that are equivalently or equivalently modified to the claims as well as the claims described below are said to belong to the scope of the spirit of the present invention.

**Claims**

1. An X-ray apparatus (10) for generating an X-ray image of an imaging region, comprising:

   an RGB camera (11) configured to generate an RGB image of the imaging region;
   a 3D camera (12) configured to measure a distance to the imaging region and generate a depth map image;
   an X-ray imager (13) configured to irradiate the imaging region and generate the X-ray image; and
   a controller (15) configured to control the X-ray imager (13),
   **characterized in that** the controller (15) is configured to:

   identify the imaging region and measure a thickness of the identified imaging region based on the RGB image and the depth map image, and
   set an exposure parameter based on the measured thickness,
   wherein the X-ray imager (13) generates the X-ray image by irradiating the imaging region using the exposure parameter set by the controller (15).

2. The X-ray apparatus (10) of claim 1, wherein the controller (15) comprises a region identifier (152) configured to identify the imaging region and an imaging angle based on the RGB image and the depth map image,
   wherein the region identifier (152) is configured to:

   identify (S320) a first imaging region and a first imaging angle from the RGB image;
   identify (S320) a second imaging region and a second imaging angle from the depth map image;
   determine (S330) whether the first imaging region and the first imaging angle are identical to the second imaging region and the second imaging angle; and
   **characterized in that,** when determined to be non-identical:

   determine (S340) one of the first imaging region and the second imaging region as the imaging region according to a preset identification priority when the identification priority is preset, and
   generate (S370) an alarm and receive an input from an operator to determine the imaging region and the imaging angle when the identification priority is not preset.

3. The X-ray apparatus (10) of claim 2, further comprising a memory (14) configured to store a region matching table in which examination regions are matched with imaging regions,

   wherein the region identifier (152) is configured to receive (S310) information about a target examination region from an operator,
   **characterized in that** the region identifier (152) is configured to:

   when the first imaging region is determined to be identical to the second imaging region, determine (S360) whether an imaging region matched to the target examination region in the region matching table is identical to the first imaging region;
   determine the first imaging region as the imaging region when the matched imaging region is identical to the first imaging region; and
   generate an alarm and receive an input from the operator when the matched imaging region is different from the first imaging region.

4. The X-ray apparatus (10) of any one of claims 1 to 3, wherein the controller (15) comprises a thickness calculator (153) configured to measure (S230) the thickness of the identified imaging region,
   **characterized in that** the thickness calculator (153) is configured to:

   detect a contour of the imaging region from the depth map image; and
   perform thickness measurement on a region inside the detected contour.

5. The X-ray apparatus (10) of claim 4, when dependent on claim 3, wherein the region matching table stored in the memory (14) further includes thickness measurement region information corresponding to each examination region,
   **characterized in that** the thickness calculator (153) is configured to measure a thickness for a partial region among regions inside the contour based on the thickness measurement region information corresponding to the target examination region.

6. The X-ray apparatus (10) of any one of claims 1, 2, 3, and 5, wherein the memory (14) stores appropriate exposure index (EI) range values matched for each imaging region and patient type,

> wherein the controller (15) further comprises a re-imaging processor (155) configured to receive the X-ray image generated by the X-ray imager (13),
> **characterized in that** the re-imaging processor (155) is configured to:
>
> > calculate (S260) an EI of the generated X-ray image; and
> > determine (S260) whether re-imaging is necessary by comparing the calculated EI with the appropriate EI range values.

7. The X-ray apparatus (10) of claim 6, wherein the memory (14) stores correction coefficients according to EI deviation values for each imaging region,

> wherein the controller (15) further comprises a parameter configurator (154),
> **characterized in that** the parameter configurator (154) is configured to:
>
> > calculate an EI deviation value between the calculated EI and the appropriate EI range values when re-imaging is determined by the re-imaging processor (155); and
> > correct (S270) the exposure parameter based on the calculated EI deviation value and the determined imaging region.

8. The X-ray apparatus (10) of any one of claims 2, 3, 5, and 7, **characterized in that** the region identifier (152) is configured to:

> identify (S320) the imaging region and the imaging angle from the RGB image and the depth map image using an artificial intelligence model trained with a plurality of pre-prepared RGB images and depth map images as input values and labels for imaging regions and imaging angles as output values,
> wherein the imaging regions and imaging angles identified by the region identifier (152) are utilized for continuous training of the artificial intelligence model.

9. A method of X-ray imaging performed by an X-ray apparatus (10) comprising an RGB camera (11), a 3D camera (12), an X-ray imager (13), and a controller (15), the method comprising:

> receiving (S210) an RGB image and a depth map image of an imaging region from the RGB camera (11) and the 3D camera (12);
irradiating the imaging region by the X-ray imager (13) to generate an X-ray image,
**characterized in that** the method further comprises:

> identifying (S220) the imaging region based on the RGB image and the depth map image;
> measuring (S230) a thickness of the identified imaging region;
> setting (S240) an exposure parameter based on the measured thickness; and
> generating (S250) the X-ray image by irradiating the imaging region using the set exposure parameter.

**FIG. 1**

# FIG. 2

Start

Receive RGB image and depth map image from RGB camera and 3D camera — 210

Identify imaging region and angle from each imaging image — 220

Measure thickness of imaging region — 230

Set exposure parameter based on measured thickness — 240

Generate X-ray Image — 250

Re-imaging necessary? — 260

No

End

Yes

Correct exposure parameter — 270

FIG. 3

EP 4 755 305 A1

13

**FIG. 4(a)**

**FIG. 4(b)**

**FIG. 5**

| Modality | Patient Type | EI Range |
|---|---|---|
| Chest X-ray | Adult | 100-300 |
| Chest X-ray | Pediatric | 50-200 |
| Abdomen X-ray | Adult | 200-400 |
| Hand X-ray | Adult | 50-150 |

**FIG. 6**

| Imaging Region | EI Deviation Value | Voltage (kV) | Current (mA) | Time (ms) |
|---|---|---|---|---|
| Chest | -15 ~ -14 | - | +0.5 | +0.2 |
|  | ... | ... | ... | ... |
|  | 1 ~ 2 | - | +0.1 | +0.06 |
|  | ... | ... | ... | ... |
|  | 15 ~ 16 | - | -0.5 | -0.2 |
| Hand | -15 ~ -14 | - | +0.2 | +0.1 |
|  | ... | ... | ... | ... |
|  | 1 ~ 2 | - | +0.07 | +0.02 |
|  | ... | ... | ... | ... |
|  | 15 ~ 16 | - | -0.2 | -0.1 |
| Foot | -15 ~ -14 | - | +0.3 | +0.2 |
|  | ... | ... | ... | ... |
|  | 1 ~ 2 | - | +0.1 | +0.08 |
|  | ... | ... | ... | ... |
|  | 15 ~ 16 | - | -0.3 | -0.2 |

<table>
<tr><td>Europäisches<br>Patentamt<br>European<br>Patent Office<br>Office européen<br>des brevets</td><td align="center">**EUROPEAN SEARCH REPORT**</td><td>**Application Number**<br><br>EP 25 22 1003</td></tr>
</table>

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2022/319030 A1 (XU YANRAN [CN] ET AL) 6 October 2022 (2022-10-06) | 1,9 | INV.<br>A61B6/00 |
| A | * paragraph [0036] - paragraph [0099] * | 2-8 | |
| | ----- | | |
| X | EP 4 052 652 A1 (KONINKLIJKE PHILIPS NV [NL]) 7 September 2022 (2022-09-07) | 1,9 | |
| A | * paragraph [0053] - paragraph [0133]; figure 2 * | 2-8 | |
| | ----- | | |
| A | EP 3 351 176 A1 (SAMSUNG ELECTRONICS CO LTD [KR]) 25 July 2018 (2018-07-25)<br>* paragraph [0028] - paragraph [0157]; figure 1 * | 1-9 | |
| | ----- | | |
| A | EP 2 944 256 A1 (SWISSRAY ASIA HEALTHCARE CO LTD [TW]) 18 November 2015 (2015-11-18)<br>* paragraph [0011] - paragraph [0033] * | 1-9 | |
| | ----- | | |

| | | |
|---|---|---|
| | | TECHNICAL FIELDS SEARCHED (IPC)<br><br>A61B |

The present search report has been drawn up for all claims

| Place of search | Date of completion of the search | Examiner |
|---|---|---|
| Munich | 21 April 2026 | Hooper, Martin |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

 

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 25 22 1003

21-04-2026

| Patent document cited in search report | Publication date | Patent family member(s) | | Publication date |
|---|---|---|---|---|
| US 2022319030 A1 | 06-10-2022 | CN | 115147430 A | 04-10-2022 |
| | | US | 2022319030 A1 | 06-10-2022 |
| EP 4052652 A1 | 07-09-2022 | CN | 116916829 A | 20-10-2023 |
| | | EP | 4052652 A1 | 07-09-2022 |
| EP 3351176 A1 | 25-07-2018 | EP | 3351176 A1 | 25-07-2018 |
| | | KR | 20180086709 A | 01-08-2018 |
| | | US | 2018206810 A1 | 26-07-2018 |
| EP 2944256 A1 | 18-11-2015 | CN | 105078499 A | 25-11-2015 |
| | | EP | 2944256 A1 | 18-11-2015 |
| | | TW | 201542170 A | 16-11-2015 |
| | | US | 2015327830 A1 | 19-11-2015 |

For more details about this annex : see Official Journal of the European Patent Office, No. 12/82

**REFERENCES CITED IN THE DESCRIPTION**

**Patent documents cited in the description**

- KR 10202400179446

- KR 1020140125502